# EUROPEAN PATENT APPLICATION

(11) **EP 4 684 750 A1**
(43) Date of publication of application: **28.01.2026**
(21) Application number: 24191148.6
(22) Date of filing: 26.07.2024
(51) Int. Cl.: A61B 34/10, A61B 18/00

(54) **FRACTAL MODEL EXTENSION FOR ABLATION ZONE CALCULATION FOR LUNG TREATMENT**

(71) Applicant: Koninklijke Philips N.V., 5656 AG Eindhoven (NL)
(72) Inventor: HENDRIKS, Bernardus Hendrikus Wilhelmus, 5656 AG Eindhoven (NL); LANGEREIS, Sander, 5656 AG Eindhoven (NL); VERSTEGE, Marco, 5656 AG Eindhoven (NL); VAN DER STERREN, William Edward Peter, 5656 AG Eindhoven (NL); ELENBAAS, Thijs, 5656 AG Eindhoven (NL)
(74) Representative: Philips Intellectual Property & Standards

(57) **Abstract**

The present invention relates ablation zone calculation for lung treatment. In order to provide improved knowledge about the distribution of the bronchi structure, a device (10) for ablation zone calculation for lung treatment is provided that comprises a data input (12), a data processor (14) and an output interface (16). The data input is configured to provide a 3D reconstruction (18) of a lung structure of a subject, the 3D reconstruction being based on image data of the subject; and to provide tissue properties (20) for a region of interest of the 3D model of the airway tree; wherein the region of interest comprises the target zone of ablation. The processor is configured to extend the 3D reconstruction with a generation of a fractal model of at least a part of an airway structure of the subject; to generate the fractal model in dependency from the 3D reconstruction, wherein the 3D reconstruction and the fractal model form a 3D model of the airway tree of the subject; to identify a target zone for ablation within the 3D model of the airway tree; to compute a thermal modelling for at least the target zone for ablation based on the 3D model of the airway tree and the tissue properties; and to predict an ablation zone (22) within the 3D model of the airway tree based on the thermal modelling. The output interface is configured to provide the predicted ablation zone to a user.

## Description

### FIELD OF THE INVENTION

The present invention relates ablation zone calculation for lung treatment. In particular, the present invention relates to a device for ablation zone calculation for lung treatment, to a system for ablation planning for lung treatment, to an ablation arrangement and to a method for ablation zone calculation for lung treatment.

### BACKGROUND OF THE INVENTION

For certain lung diseases, e.g. lung cancer, tissue ablation can be applied for treatment purposes. For ablation, energy such as thermal energy is applied to target tissue structure in order to affect the tissue, e.g. to destroy the tissue. Ablation can be achieved by applying heat to destroy tumorous cells. However, ablation can also be provided by cooling, i.e. by applying cold temperatures. Ablation of lung lesion may be hampered by the presence of air in the bronchi, and alveoli in the lung. In addition, blood flow may cause a heat sink effect. This heat sink effect is a challenge for thermal ablation techniques where blood flow or airflow through the target tissue can offset the intended heating or cooling, affecting, e.g. limiting, tissue damage, i.e. affecting the ablation. Thermal conductivity is another factor that impacts thermal ablation. Lung tissue has lower thermal conductivity than bone, liver, and kidney, which can inhibit heat transfer to tissues adjacent to solid pulmonary masses. This could lead to inadequate ablation of infiltrative margins or satellite tumors at the periphery of lung lesions. Although air-filled lung tissue may restrict thermal, e.g. heat, transfer to the partially aerated peripheral portion of lesions, it can also insulate the interior of lesions and improve tumor damage through an oven/freezer-type effect. In order to predict the ablation zone that can be created in lung tissue, anatomical knowledge of the lung near the tumor is important. As an example, computed tomography (CT) or cone beam computed tomography (CBCT) X-ray imaging is used to reconstruct a part of the airway tree. However, it has been shown that the deeper structures are too small to be reconstructed. As a result, to predict the reached final ablation, cannot accurately be made.

### SUMMARY OF THE INVENTION

There may thus be a need for improved knowledge about possible ablation results.

The object of the present invention is solved by the subject-matter of the independent claims; further embodiments are incorporated in the dependent claims. It should be noted that the following described aspects of the invention apply also for the device for ablation zone calculation for lung treatment, for the system for ablation planning for lung treatment, for the ablation arrangement and for the method for ablation zone calculation for lung treatment.

According to the present invention, a device for ablation zone calculation for lung treatment is provided. The device comprises a data input, a data processor and an output interface. The data input is configured to provide a 3D reconstruction of a lung structure of a subject; the 3D reconstruction being based on image data of the subject. The data input is also configured to provide tissue properties for a region of interest of the 3D model of the airway tree; the region of interest comprises a target zone of ablation. The data processor is configured to extend the 3D reconstruction with a generation of a fractal model of at least a part of an airway structure of the subject. The data processor is also configured to generate the fractal model in dependency from the 3D reconstruction. The 3D reconstruction and the fractal model form a 3D model of the airway tree of the subject. The data processor is further configured to identify the target zone for ablation within the 3D model of the airway tree. The data processor is furthermore configured to compute a thermal modelling for at least the target zone for ablation based on the 3D model of the airway tree and the tissue properties, and to predict an ablation zone within the 3D model of the airway tree based on the thermal modelling. The output interface is configured to provide the predicted ablation zone to a user.

As an effect, the prediction of the ablation zone when treating lung nodules is improved.

According to an example, to calculate the heat distribution of the ablation, the data input is configured to provide an input of the location the heat is coming from as a starting point.

In an option, this is an input from the user in the form of a needle/device planning, e.g. represented by 3D line indicating tip and orientation, or a 3D scan with an already placed device, and e.g. potentially the specifications of the device. In this last case the system may automatically detect the device position.

In another option, an algorithm determines an optimal positioning of the device.

As an example, in case the user cannot place the device(s) exactly as planned, the predicted ablation zone on the positioned device is provided.

According to an example, the data processor is configured to grow the fractal model from the 3D reconstruction. The data processor is configured to use end portions of airway branches of the 3D reconstruction as input starting points for the generating of the fractal model.

This provides the advantage that due to using the 3D reconstruction as a base for growing the fractal model, the basic structures are subject specific. Growing the fractal model from the end portions of the 3D reconstruction reduces the computing effort for the fractal model growing process.

According to an example, for identification of the end portions, the data processor is configured to identify at least one anatomic detail of the group comprising the trachea, the primary bronchi, the secondary bronchi and the tertiary bronchi and to determine as at least one start portion of the 3D reconstruction lung structure. The data processor is configured to identify ends of the 3D reconstruction lung structure pathways as the end portions in a distal relation to the at least one start portion.

According to an example, for an extension of the 3D reconstruction, the data processor is configured to generate a plurality of fractal model segments. The 3D reconstruction and the fractal model segments form the 3D model of the airway tree.

According to an example, for an extension of the 3D reconstruction, the data processor is configured to generate a fractal model of the lung structure, and to adapt the fractal model to the 3D reconstruction to form the 3D model of the airway tree. For the adaptation, the data processor is configured to register the fractal model to the 3D reconstruction starting from a source point of the fractal model.

According to an example, for the provision of the 3D reconstruction, the data input is configured to provide 3D X-ray image data, and the data processor is configured to compute the 3D reconstruction of the lung structure of the subject based on the 3D X-ray image data.

According to an example, for a computation of the thermal modelling, the data input is configured to provide data of an ablation device to be used for the ablation. The data processor is configured to base the thermal modelling on the tissue properties and on the data of the ablation device.

According to an example, the data input is configured to provide tumor information. The data processor is configured to generate a representation of the tumor. The output interface us configured to show the representation of the tumor and the ablation zone in spatial relation to each other.

To provide tumor information is optional. In an example, the user identifies the tumor. In another example, the system recognizes the tumor from the volume data and the provided device location(s).

According to an example, the output interface is configured to provide the ablation zone with at least two graduations relating to a degree of predicted ablation comprising at least: predicted necrotic tissue, and predicted heated tissue that can regenerate.

In another option, just the predicted necrotic part is shown, i.e. the part that after ablation is no longer viable tissue.

According to the present invention, also a system for ablation planning for lung treatment is provided. The system comprises an imaging device and an example of the device for ablation zone calculation for lung treatment according to one of the preceding examples. The imaging device is configured to provide the image data of the subject for computing the 3D reconstruction of the lung structure of the subject.

According to the present invention, also an ablation arrangement is provided, The arrangement comprises an example of a device for ablation zone calculation for lung treatment according to one of the example above, or an example of the system for ablation planning for lung treatment according to the preceding example. The arrangement also comprises an ablation device. The ablation device is configured to provide an ablation to the target zone for ablation according to the predicted ablation zone.

According to the present invention, also a method for ablation zone calculation for lung treatment is provided. The method comprises the following steps:
- Providing a 3D reconstruction of a lung structure of a subject, the 3D reconstruction being based on image data of the subject;
- Extending the 3D reconstruction with a generating of a fractal model of at least a part of an airway structure of the subject; the fractal model is generated in dependency from the 3D reconstruction; and the 3D reconstruction and the fractal model are forming a 3D model of the airway tree of the subject;
- Identifying a target zone for ablation within the 3D model of the airway tree.
- Providing tissue properties for a region of interest of the 3D model of the airway tree; the region of interest comprising the target zone of ablation;
- Computing a thermal modelling for at least the target zone for ablation based on the 3D model of the airway tree and the tissue properties;
- Predicting an ablation zone within the 3D model of the airway tree based on the thermal modelling; and
- Providing the ablation zone to a user.

According to an aspect, a more precise lung airway tree reconstruction with corresponding tissue properties is provided. This is then used in thermal modelling to predict the ablation zone. To be able to determine the lung structures near the ablation area, first a CBCT is made from which the larger structures of the airways and the location of the lung lesion are reconstructed. Next, the remaining part of the lung is modelled using a fractal model of the lung. The already reconstructed part is extended. Next, for the location of the lesion, the structure of the lung is extracted from the reconstructed lung. This composition is used in the thermal model to predict the ablation zone that will be reached by the ablation device.

In an example, from a 3D cone beam CT taken during the intervention, the 3D airways model is reconstructed. As an option, the trachea is identified first, and from that the airway tree is grown by inflation. In this way the first few bronchi can be reconstructed.

Further, the airway tree that is known from the CBCT airway reconstruction is extended based on an algorithm for reconstructing a 3D model of the human airways. As a result, a better estimation of the lung tissue distribution near the lung lesion that will be ablated is obtained.

As an option, the reconstructing of the 3D model of the human airways is based on a deterministic algorithm that incorporates both duct branching and space division. An example is the algorithm developed by Hiroko Kitaoka et al.. The algorithm operates based on two principles: Firstly, the volume of region supplied by a branch is directly proportional to the amount of fluid it delivers. Secondly, the terminal branches are uniformly distributed in the organ. These principles determine the fundamental branching process: the dimensions and direction of daughter branches are determined by the characteristics of the parent branch and the provided region. The algorithm consists of nine fundamental rules and four additional rules. Once the trunk position and organ contour are specified, the algorithm produces branches successively. When applied, the algorithm creates an airway tree containing typically over 54000 branches of the human lung.

According to the invention, in an option, the algorithm is modified and applied in a multi-fold manner starting from the plurality of airway segments identified by the CBCT airway reconstruction. As a result, an enhanced airway tree reconstruction is achieved.

From the airway tree reconstruction, the ratio of air (related to the bronchi in the ablation volume) and blood (linked to the blood vessels that run along the airway tree) is determined, as well as the rest of the lung tissue. For each of these the thermal tissue properties are known.

Once the tissue distribution is known near the lung lesion, a thermal modelling of the ablation zone is conducted with thermal modelling software. As a result, the final zone is computed.

According to the invention, in another option, the algorithm is used to generate one fractal airway model.

According to the invention, in a further option, the algorithm is used to generate an ensemble of models that are used and the averaged structure is calculated for determining the ratio of air and blood.

In an option, from the CBCT, a structure of the lesion is determined, for instance using a trained neural network to determine the tissue properties needed for thermal modelling.

In another option, a neural network is used with a hidden physical model to determine an ablation zone instead of thermal modelling.

An example for a field of use are X-ray guided lung ablation procedures in the cathlab.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for ablation zone calculation for lung treatment.
Fig. 2 shows an example of a system for ablation planning for lung treatment. As an option, an ablation arrangement is also shown in the context of an operation room.
Fig. 3 shows basic steps of an example of a method for ablation zone calculation for lung treatment.
Fig. 4 shows an example of a working scheme for ablation zone calculation for lung treatment.
Fig. 5 shows an example of a lung airway reconstruction based on CT lung images.
Figs. 6a-6d schematically illustrate steps of an ablation zone calculation for lung treatment. Fig. 6a indicates image data provided on the right, and a lung airway reconstruction model based on the lung images on the left. Fig. 6b indicates an extension of the lung airway reconstruction model by combining it with fractal model parts. Fig. 6c illustrates an example of a thermal modelling. Fig. 6d shows an illustration of a simulated result.

### DETAILED DESCRIPTION OF THE EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

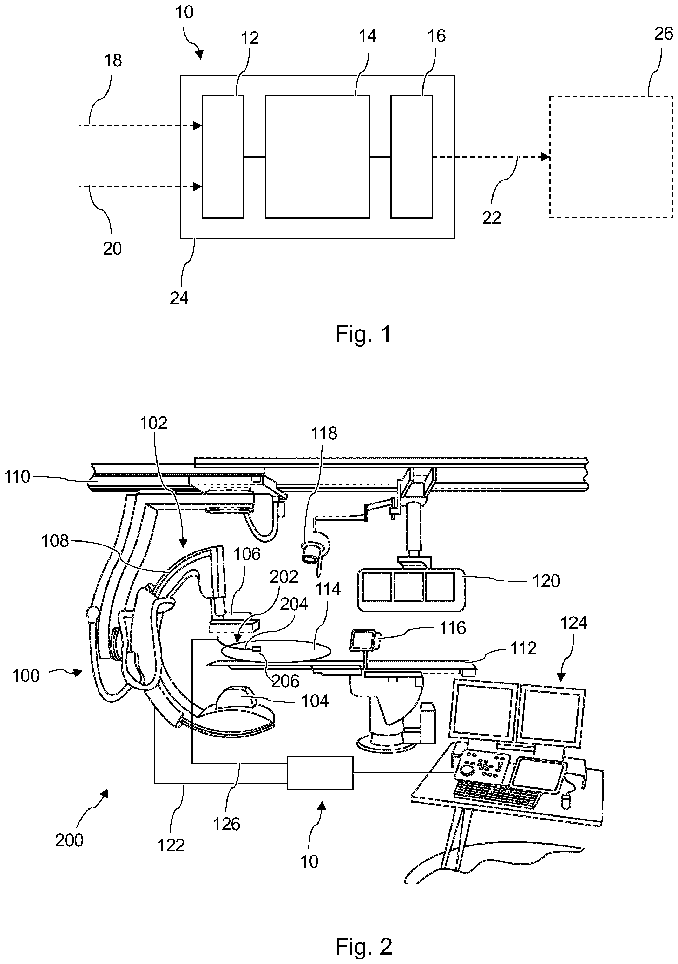

Fig. 1 schematically shows an example of a device 10 for ablation zone calculation for lung treatment. The device 10 comprises a data input 12, a data processor 14 and an output interface 16. The data input 12 is configured to provide a 3D reconstruction of a lung structure of a subject, the 3D reconstruction being based on image data of the subject. The data input 12 is also configured to provide tissue properties for a region of interest of the 3D model of the airway tree. The region of interest comprises the target zone of ablation. The data processor 14 is configured to extend the 3D reconstruction with a generation of a fractal model of at least a part of an airway structure of the subject. The data processor 14 is further configured to generate the fractal model in dependency from the 3D reconstruction. The 3D reconstruction and the fractal model form a 3D model of the airway tree of the subject. The data processor 14 is furthermore configured to identify a target zone for ablation within the 3D model of the airway tree. The data processor 14 is also configured to compute a thermal modelling for at least the target zone for ablation based on the 3D model of the airway tree and the tissue properties. Still further, the data processor 14 is configured to predict an ablation zone within the 3D model of the airway tree based on the thermal modelling. The output interface 16 is configured to provide the predicted ablation zone to a user.

A first arrow 18 indicates the provision of the 3D reconstruction. A second arrow 20 indicates the provision of the tissue properties. A third arrow 22 indicates the provision of the predicted ablation zone.

A first frame 24 indicates the arrangement of the data input 12, the data processor 14 and the output interface 16 in a common structure, e.g. in an integrated manner. However, the data input 12, the data processor 14 and the output interface 16 can also be arranged separated.

A second frame 26 indicates a display that is shown as an option. The predicted ablation zone can be shown on the display.

The term "3D reconstruction of a lung structure" relates to a representation of the lung and its structure in 3D based on image data of the subject. As an example, X-ray CT imaging is provided, and the lung is reconstructed based on the CT data. As another example, further image data sources like ultrasound imaging or MR imaging are provided.

In an example, X-ray imaging is used for acquiring the 3D image data of the subject.

The X-ray image data is providing information about the current subject situation.

In an option, a thorax shape is retrieved from the X-ray image data.

In another option, the thorax vertebrae are identified based on the X-ray image data.

In an option, a display is provided to present the ablation zone to a user.

The term "tissue properties" relates to characteristics of the particular tissue part. The properties are relating to ablation procedures, like heat transmission or heat dissipation.

The term "based on image data" relates to the image data being an essential part of the computing steps.

The term "target zone of ablation" relates to a planned zone for applying the ablation.

The term "ablation" relates to a procedure in which tissue is affected, e.g. destroyed. As an example, in an endobronchial device, or one or more needles are used to harm the tissue in such a way, with the goal to destroy tumor cells. Different technologies are used: radiofrequency, microwave, laser, high-intensity focused ultrasound, cryoablation, and irreversible electroporation.

The term "thermal modelling" relates to simulating heat or other thermal energy supply and its distribution and effects, e.g. based on experimental data.

The term "ablation zone" relates to the actual zone in which the ablation with the chosen settings is effective. The ablation zone can also be referred to as ablation affected zone.

The term "data input" relates to providing or supplying data for data processing steps. The data input 12 can also be referred to as image data input. The data input 12 can also be referred to as data supply, as image data supply, as image input, as input unit or simply as input. In an example, the image data input 12 is data-connectable to an imaging source arrangement.

The term "data processor" relates to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input and the output interface.

The term "output interface" relates to an interface for providing the processed or computed data for further purposes. The output interface 16 can also be referred to as output or output unit. In an example, the output interface 16 is data-connectable to a display arrangement or display device. In another example, the output interface 16 is data-connected to a display. As an example, the signals by the controller can be provided by the output interface 16.

In an example, to calculate the heat distribution of the ablation, the data input 12 is configured to provide an input of the location the heat is coming from as a starting point.

In an option of the example of Fig. 1, the data processor 14 is configured to grow the fractal model from the 3D reconstruction. The data processor 14 is also configured to use end portions of airway branches of the 3D reconstruction as input starting points for the generating of the fractal model.

The term "to grow" relates to the mathematical generation of 3D data when starting from a defined originating point.

The term "end portions" relates to parts of the 3D reconstruction where a lumen ends due to image data being not provided for this region, e.g. because the image resolution does not allow finer details.

In another option of the example of Fig. 1, for identification of the end portions, the data processor 14 is configured to identify at least one anatomic detail of the group comprising the trachea, the primary bronchi, the secondary bronchi and the tertiary bronchi and to determine as at least one start portion of the 3D reconstruction lung structure. The data processor 14 is also configured to identify ends of the 3D reconstruction lung structure pathways as the end portions in a distal relation to the at least one start portion.

The term "start portion" relates to where a branch or pathway starts in terms of the treelike arrangements of the lung structures.

In an example, the at least one anatomic detail of the group comprising the trachea, the primary bronchi, the secondary bronchi and the tertiary bronchi is identified based on X-ray image data.

In an option of the example of Fig. 1, for an extension of the 3D reconstruction, the data processor 14 is configured to generate a plurality of fractal model segments. The 3D reconstruction and the fractal model segments are forming the 3D model of the airway tree.

The 3D model can also be referred to as 3D representation.

The term "fractal model segments" relates to separate fractal branch portions.

In a further option of the example of Fig. 1, for an extension of the 3D reconstruction, the data processor 14 is configured to generate a fractal model of the lung structure. The data processor 14 is also configured to adapt the fractal model to the 3D reconstruction to form the 3D model of the airway tree. For the adaptation, the data processor 14 is configured to register the fractal model to the 3D reconstruction starting from a source point of the fractal model.

The 3D model can be segmented into different lobes, for which different colors can be applied to facilitate a user's orientation.

According to an aspect, a 3D X-ray model is extended by model generating procedures applied to those parts of the 3D X-ray model where details are to be expected.

In an option of the example of Fig. 1, for the provision of the 3D reconstruction, the data input 12 is configured to provide 3D X-ray image data, and the data processor 14 is configured to compute the 3D reconstruction of the lung structure of the subject based on the 3D X-ray image data.

In an option of the example of Fig. 1, for an identification of the target zone for ablation, the data processor 14 is configured to detect a lesion in the 3D reconstruction or the image data.

The term "lesion" relates to e.g. an identified tumor or other structures.

In an option of the example of Fig. 1, for a computation of the thermal modelling, the data input 12 is configured to provide data of an ablation device to be used for the ablation. The data processor 14 is configured to base the thermal modelling on the tissue properties and on the data of the ablation device.

The term "data of an ablation device" relates to characteristics of the device itself and also the application details.

In another option of the example of Fig. 1, the data input 12 is configured to provide tumor information. The data processor 14 is configured to generate a representation of the tumor. The output interface 16 is configured to show the representation of the tumor and the ablation zone in spatial relation to each other.

The term "representation" relates to an indicator or simulation of the tumor or other cancerous structures.

The term "spatial relation" relates to e.g. position and size in 3D.

In an option, a display is provided to present the representation of the tumor and the ablation zone in spatial relation to each other to a user.

The representation of the tumor and the ablation zone are shown in context with each other. In an example, the representation of the tumor is overlaid to the ablation zone. In another example, the ablation zone is overlaid to the representation of the tumor.

In an example, the detected lesion is used to generate the tumor information.

In an option of the example of Fig. 1, the data processor 14 is configured to correct the data of the ablation device based on changing at least one parameter of the planned ablation procedure. The data processor 14 is configured to compute an adapted thermal modelling to provide an adapted ablation zone.

In an option, an interface for adapting or changing the ablation device data is provided. For example, a user can select a different ablation tool from a number of tools. In another example, a user can change settings of an ablation tool.

This allows to tailor the ablation procedure to an optimum ablation, avoiding i) to ablate too much lung tissue or ii) to ablate too little lung tissue.

In an option of the example of Fig. 1, the output interface 16 is configured to provide the ablation zone with at least two graduations relating to a degree of predicted ablation. The degrees of predicted ablation are comprising at least predicted necrotic tissue and predicted heated tissue that can regenerate.

The term "necrotic tissue" relates to tissue that is destroyed. In the ablation process, the device will only heat up or freeze the tissue such that the cells in the tissue are no longer viable (hence function no longer). These cells are necrotic.

In an example, the body itself detects these cells and reabsorbs these partially.

In a further example, the destroyed tissue is partially reabsorbed by the body, and some parts may remain.

In a further example, the destroyed tissue is partially actively removed and partly absorbed.

In an example, the destroyed tissue is not actively removed.

In another possible example, the destroyed tissue is removed.

The term "heated tissue that can regenerate" relates to tissue that may recover from the thermal application.

In another option, at least one further graduation is provided for indicating tissue that is warmed up but not heated.

In an option, a display is provided to present the ablation zone with graduations, such as different colors.

Fig. 2 shows an example of a system 100 for ablation planning for lung treatment. The system 100 comprises an example of the device 10 for ablation zone calculation for lung treatment according to one of the preceding examples. The system also comprises an imaging device 102. The imaging device 102 is configured to provide the image data of the subject for computing the 3D reconstruction of the lung structure of the subject.

As an example, the imaging system 102 is an X-ray imaging device with an X-ray source 104 and an X-ray detector 106 mounted to a movable C-arm 108. The C-arm is suspended from a ceiling rail system 110. Further, as an option, a subject support 112 is provided. A subject 114 is indicated being arranged on the subject support 112. A bedside controller 116 can be arranged next to the subject support 112. Still further, adaptable lighting 118 can be provided. Furthermore, a display arrangement 120 is also provided. A first connection line 122 indicates a data-connection wireless or wire-bound, between the imaging system 102 and the device 10 for ablation zone calculation for lung treatment.

As an option, an operating console 124 is indicated for controlling the various equipment.

As an option, Fig. 2 also shows an example of an ablation arrangement 200 in the context of the system 100 for ablation planning for lung treatment. The ablation arrangement 200 comprises an ablation device 202. In a first variation, the ablation arrangement 200 comprises an example of the device 10 for ablation zone calculation for lung treatment according to one of the examples above. In a second variation, the ablation arrangement 200 comprises an example of the system 100 for ablation planning for lung treatment according to the examples above. The ablation device 202 is configured to provide an ablation to the target zone for ablation according to the predicted ablation zone.

The ablation device 202 is indicated with an elongate body 204, like a catheter, at least partly insertable into e.g. airways of the subject 114. A treatment component 206 can be arranged on the distal tip of the ablation device 202.

A second connection line 126 indicates a data-connection wireless or wire-bound, between the ablation device 202 and the device 10 for ablation zone calculation for lung treatment.

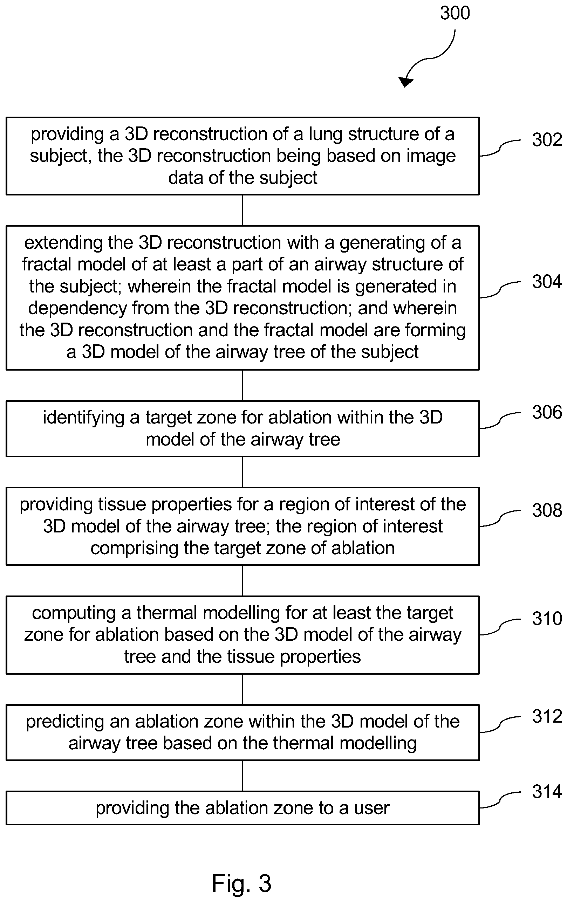

Fig. 3 shows basic steps of an example 300 of a method for ablation zone calculation for lung treatment. The method 300 comprises the following steps:
- In a first step 302, a 3D reconstruction of a lung structure of a subject is provided. The 3D reconstruction is based on image data of the subject.
- In a second step 304, the 3D reconstruction is extended by generating a fractal model of at least a part of an airway structure of the subject. The fractal model is generated in dependency from the 3D reconstruction. The 3D reconstruction and the fractal model are forming a 3D model of the airway tree of the subject.
- In a third step 306, a target zone for ablation within the 3D model of the airway tree is identified.
- In a fourth step 308, tissue properties for a region of interest of the 3D model of the airway tree are provided. The region of interest comprises the target zone of ablation.
- In a fifth step 310, a thermal modelling is computed for at least the target zone for ablation based on the 3D model of the airway tree and the tissue properties.
- In a sixth step 312, an ablation zone within the 3D model of the airway tree is predicted based on the thermal modelling.
- In a seventh step 314, the ablation zone is provided to a user.

The fifth step 310 can also be provided in a different order, but before the sixth step 312.

The method for ablation zone calculation for lung treatment can also be referred to as method for lung treatment ablation zone calculation.

In an example of the method, the fractal model is grown from the 3D reconstruction. Further, end portions of airway branches of the 3D reconstruction are used as input starting points for the generating of the fractal model.

In an example of the method, for identifying the end portions, at least one anatomic detail of the group comprising the trachea, the primary bronchi, the secondary bronchi and the tertiary bronchi is identified and determined as at least one start portion of the 3D reconstruction lung structure. Further, ends of the 3D reconstruction lung structure pathways distal to the at least one start portion are identified as the end portions.

In an example of the method, the extending of the 3D reconstruction comprises generating a plurality of fractal model segments. Further, the 3D reconstruction and the fractal model segments are forming the 3D model of the airway tree.

In an example of the method, the extending of the 3D reconstruction comprises generating a fractal model of the lung structure. Further, the fractal model is adapted to the 3D reconstruction to form the 3D model of the airway tree. Still further, for adapting, the fractal model is registered to the 3D reconstruction starting from a source point of the fractal model.

In an example of the method, for providing the 3D reconstruction, 3D X-ray image data is provided, and the 3D reconstruction of the lung structure of the subject is computed based on the 3D X-ray image data.

In an example of the method, for identifying the target zone for ablation, a lesion is detected in the 3D reconstruction or the image data.

In an example of the method, for computing the thermal modelling, data of an ablation device to be used for the ablation is provided. Further, the thermal modelling is based on the tissue properties and based on the data of the ablation device.

In an example of the method, tumor information is provided, and a representation of the tumor is generated. Further, the representation of the tumor and the ablation zone are shown in spatial relation to each other.

In an example of the method, the data of the ablation device is corrected based on changing at least one parameter of the planned ablation procedure. Further, an adapted thermal modelling is computed to provide an adapted ablation zone.

In an example of the method, the ablation zone is provided with at least two graduations relating to a degree of predicted ablation comprising at least: predicted necrotic tissue, and predicted heated tissue that can regenerate.

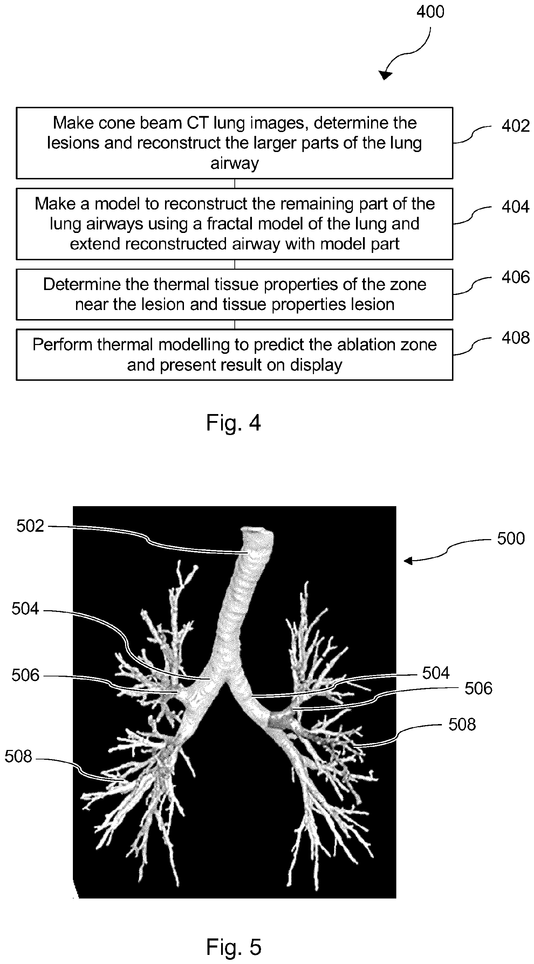

Fig. 4 shows an example of a working scheme 400 for ablation zone calculation for lung treatment. In a first scene 402, cone beam CT lung images are provided, e.g. acquired. The lesions are determined and the larger parts of the lung airway are reconstructed. In a second scene 404, a model is made to reconstruct the remaining part of the lung airway using a fractal model of the lung. The reconstructed airway is thus extended with the fractal model part. In a third scene 406, the thermal tissue properties of the zone near the lesion are determined. Further, also the tissue properties of the lesion are determined. In a fourth scene 408, thermal modelling is performed to predict the ablation zone. The result is present on a display.

The term "scene" relates to a processing or action step that may also comprise several sub-steps or smaller tasks.

Fig. 5 shows an example of a lung airway reconstruction 500 based on CT lung images. As a main part, a trachea 502 is forming a base from which primary bronchi 504 branch off. Further, secondary bronchi 506 and the tertiary bronchi 508 are also identified.

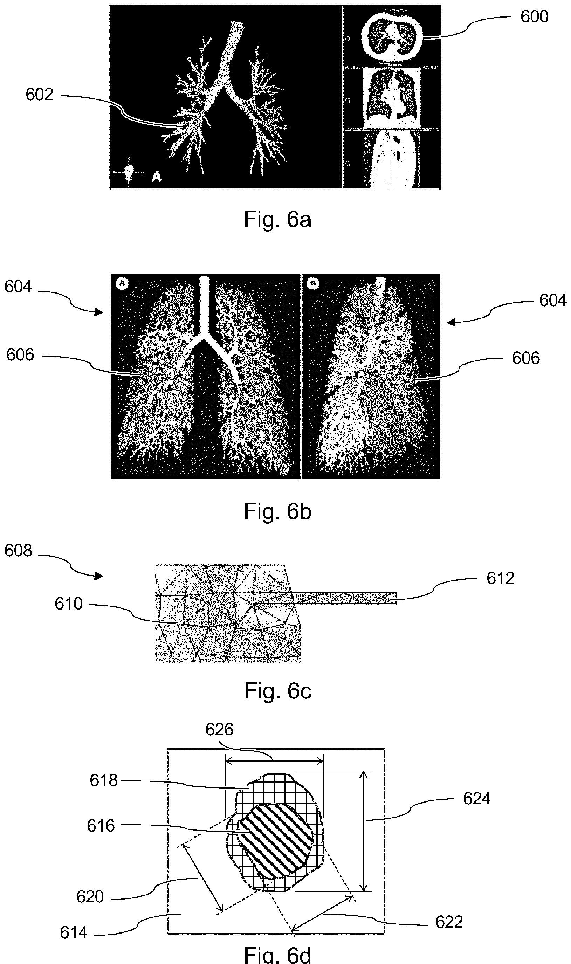

Figs. 6a-6d schematically illustrate steps of an ablation zone calculation for lung treatment.

Fig. 6a indicates image data 600 provided on the right and a lung airway reconstruction model 602 (see Fig. 5) on the left based on the lung images. The lung airway reconstruction model 602 already provides a representation of major parts of the airways, but not the finer airway structures.

Fig. 6b indicates an extension 604 of the lung airway reconstruction model by combining fractal model parts 606. The fractal parts so-to-speak complete the lung airway reconstruction model 602. The left part shows the lung representation in a frontal view; the right part shows the lung representation in a sagittal view from the left.

Fig. 6c illustrates an example of a thermal modelling 608. A tissue structure is indicated in the left part with a mesh 610. A device 612 is reaching into the tissue from the right. Different pattern or colors (not shown in detail) can be used for illustrating the different temperature predictions.

Fig. 6d shows an illustration of a simulated result. A lung structure 614 is shown with a central part 616 subject to a device position and thus a predicted first effect, e.g. destroying the tissue with cold or heat. A surrounding portion 618 indicates a transition zone in which temperature affects the tissue, but only to such an extent, where a regeneration can be expected. A first scale 620 indicates a maximum size of the central part 616 and a second scale 622 indicates a minimum size of the central part 616. A third scale 624 indicates a maximum size of the surrounding portion 618 and a fourth scale 626 indicates a minimum size of the surrounding portion 618.

The term "subject" may also be referred to as individual. The "subject" may further also be referred to as patient, although it is noted that this term does not indicate whether any illness or disease is actually present with the subject.

In an example, a computer program is provided comprising instructions which, when the program is executed by a computer, cause the computer to carry out the method of one of the examples above.

In an example, a computer readable medium having stored the computer program of the previous example is provided.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for ablation zone calculation for lung treatment, the device comprising:
- a data input (12);
- a data processor (14); and
- an output interface (16);
wherein the data input is configured: to provide a 3D reconstruction (18) of a lung structure of a subject, the 3D reconstruction being based on image data of the subject; and to provide tissue properties (20) for a region of interest of the 3D model of the airway tree; wherein the region of interest comprises a target zone of ablation;
wherein the data processor is configured: to extend the 3D reconstruction with a generation of a fractal model of at least a part of an airway structure of the subject; to generate the fractal model in dependency from the 3D reconstruction, wherein the 3D reconstruction and the fractal model form a 3D model of the airway tree of the subject; to identify the target zone for ablation within the 3D model of the airway tree; to compute a thermal modelling for at least the target zone for ablation based on the 3D model of the airway tree and the tissue properties; and to predict an ablation zone (22) within the 3D model of the airway tree based on the thermal modelling; and
wherein the output interface is configured to provide the predicted ablation zone to a user.

2. Device according to claim 1, wherein, to calculate the heat distribution of the ablation, the data input is configured to provide an input of the location the heat is coming from as a starting point.

3. Device according to claim 1 or 2, wherein the data processor is configured to grow the fractal model from the 3D reconstruction; and
wherein the data processor is configured to use end portions of airway branches of the 3D reconstruction as input starting points for the generating of the fractal model.

4. Device according to claim 1, 2 or 3, wherein, for identification of the end portions, the data processor is configured to identify at least one anatomic detail of the group comprising the trachea, the primary bronchi, the secondary bronchi and the tertiary bronchi and to determine as at least one start portion of the 3D reconstruction lung structure; and
wherein the data processor is configured to identify ends of the 3D reconstruction lung structure pathways as the end portions in a distal relation to the at least one start portion.

5. Device according to one of the preceding claims, wherein for an extension of the 3D reconstruction, the data processor is configured to:
i) generate a fractal model of the lung structure; and to adapt the fractal model to the 3D reconstruction to form the 3D model of the airway tree; wherein, for the adaptation, the data processor is configured to register the fractal model to the 3D reconstruction starting from a source point of the fractal model; or
ii) generate a plurality of fractal model segments; wherein the 3D reconstruction and the fractal model segments form the 3D model of the airway tree.

6. Device according to one of the preceding claims, wherein, for the provision of the 3D reconstruction, the data input is configured to provide 3D X-ray image data, and the data processor is configured to compute the 3D reconstruction of the lung structure of the subject based on the 3D X-ray image data.

7. Device according to one of the preceding claims, wherein, for an identification of the target zone for ablation, the data processor is configured to detect a lesion in the 3D reconstruction or the image data.

8. Device according to one of the preceding claims, wherein, for a computation of the thermal modelling, the data input is configured to provide data of an ablation device to be used for the ablation; and
wherein the data processor is configured to base the thermal modelling on the tissue properties and on the data of the ablation device.

9. Device according to one of the preceding claims, wherein the data input is configured to provide tumor information; and the data processor is configured to generate a representation of the tumor; and
wherein the output interface us configured to show the representation of the tumor and the ablation zone in spatial relation to each other.

10. Device according to one of the preceding claims, wherein the data processor is configured to correct the data of the ablation device based on changing at least one parameter of the planned ablation procedure; and
wherein the data processor is configured to compute an adapted thermal modelling to provide an adapted ablation zone.

11. Device according to one of the preceding claims, wherein the output interface is configured to provide the ablation zone with at least two graduations relating to a degree of predicted ablation comprising at least:
- predicted necrotic tissue; and
- predicted heated tissue that can regenerate.

12. A system (100) for ablation planning for lung treatment, the system comprising:
- a device (10) for ablation zone calculation for lung treatment according to one of the preceding claims; and
- an imaging device (102);
wherein the imaging device is configured to provide the image data of the subject for computing the 3D reconstruction of the lung structure of the subject.

13. An ablation arrangement (200), comprising:
- a device (10) for ablation zone calculation for lung treatment according to one of the claims 1 to 11; or a system (100) for ablation planning for lung treatment according to claim 12; and
- an ablation device (202);
wherein the ablation device is configured to provide an ablation to the target zone for ablation according to the predicted ablation zone.

14. A method (300) for ablation zone calculation for lung treatment, the method comprising the following steps:
- providing (302) a 3D reconstruction of a lung structure of a subject, the 3D reconstruction being based on image data of the subject;
- extending (304) the 3D reconstruction with a generating of a fractal model of at least a part of an airway structure of the subject; wherein the fractal model is generated in dependency from the 3D reconstruction; and wherein the 3D reconstruction and the fractal model are forming a 3D model of the airway tree of the subject;
- identifying (306) a target zone for ablation within the 3D model of the airway tree;
- providing (308) tissue properties for a region of interest of the 3D model of the airway tree; the region of interest comprising the target zone of ablation;
- computing (310) a thermal modelling for at least the target zone for ablation based on the 3D model of the airway tree and the tissue properties;
- predicting (312) an ablation zone within the 3D model of the airway tree based on the thermal modelling; and
- providing (314) the ablation zone to a user.

15. A computer program comprising instructions which, when the program is executed by a computer, cause the computer to carry out the method of claim 14.
